# EUROPEAN PATENT APPLICATION

(11) **EP 2 574 916 A2**
(43) Date of publication of application: **03.04.2013**
(21) Application number: 12186366.6
(22) Date of filing: 27.09.2012
(51) Int. Cl.: G01N 29/07, G01N 33/38

(54) **Quality control of concrete vibration**

(30) Priority: 28.09.2011 US 201161540199 P; 20.09.2012 US 201213623345
(71) Applicant: Electric Power Research Institute, Inc., Charlotte, NC 28262 (US)
(72) Inventor: Le Pape, Yann Marcel Rene, Huntersville, North Carolina (US); Biagini, Maria Silvia Guimaraes, Charlotte, North Carolina 28211 (US); Lindberg, John T., Huntersville, North Carolina 28078 (US); Wall, James Joseph, Charlotte, North Carolina 28205 (US)
(74) Representative: Brandon, Paul Laurence

(57) **Abstract**

An apparatus (10, 20) and method of controlling concrete vibration is disclosed. The apparatus (10, 20) includes a global positioning sensor (11, 21) adapted to provide a location of a vibrator relative to a concrete structure, a recording device (21, 22) adapted to record data during vibration of the concrete, and a data transmission device (13, 24) adapted to transmit the data to a processor (14, 26) for analysis of the data to determine if the vibration is in compliance. The vibrator is adapted to vibrate the concrete to remove air contained therein and promote concrete consolidation.

## Description

### BACKGROUND OF THE INVENTION

This application relates to an apparatus and method for controlling the quality of concrete and, more particularly, to an apparatus and method of controlling concrete vibration.

Concrete structures, such as nuclear facilities, often use thick and/or large sections of concrete to form the appropriate structure. These types of structures require that the concrete forming the structures be of a high quality, resulting in quality control challenges. Two such challenges are the quality of concrete vibration and the placement of the concrete.

Poor quality control of concrete vibration and placement can result in low concrete quality which compromises the durability of the structures and creates delays and cost overruns in construction. One of the problems during the construction of concrete structures is degradation of the concrete due to aging. In nuclear constructions, these problems are described in NUREG 4652 (existing fleet) and NRC IN 2008-17 (new plants in other countries).

The acceptance criterions for new construction are mainly based on visual inspection performed by an inspector on site. Quality Control is performed visually by an inspector on site that has the task of ensuring that the proper vibration procedures are followed, resulting in excessive responsibility for the inspectors. Common imperfections resulting from under-vibration are honeycombing, excessive amounts of entrapped air voids, and layer lines. When hidden in the concrete, mass flaws like these are hardly detectable and when detected later, as the structure ages, become the responsibility of the owner.

Correct placement of concrete requires consolidation generally achieved by internal vibrators for normal weight concrete. Vibration ensures that the entrapped air contained in unconsolidated concrete is released leading to a volume change of about 5% to 20%. Under-vibration is a frequent cause of poor consolidation. It is commonly accepted that under-vibration is far more common than over-vibration. Under-vibration may be caused by the following: (1) use of an undersized, underpowered, or poorly maintained vibrator; (2) excessive or haphazard spacing of vibrator insertions; (3) inadequate vibration during each insertion; (4) failure of the vibrator to penetrate into the preceding layer; and/or (5) vibrator in the wrong position relative to the form. Currently, there are no available concrete vibrators that include quality control and that can track the location and duration of the vibration process.

### BRIEF SUMMARY OF THE INVENTION

Accordingly, there is a need for an apparatus and method that includes quality control and has the ability to track the location and duration of the vibration process.

According to one aspect of the invention, an apparatus for ensuring proper concrete consolidation includes a global positioning sensor adapted to provide a location of a vibrator relative to a concrete structure, a recording device adapted to record data during vibration of the concrete, and a data transmission device adapted to transmit the data to a processor for analysis of the data to determine if the vibration is in compliance. The vibrator is adapted to vibrate the concrete to remove air contained therein and promote concrete consolidation.

The invention also includes an apparatus for ensuring proper concrete consolidation includes a vibrator for vibrating concrete to promote proper concrete consolidation and a quality control unit connected to the vibrator. The quality control unit includes a global positioning sensor adapted to provide a location of the vibrator relative to a concrete structure to be vibrated, a recording device adapted to record data during vibration of the concrete, and a transmission device adapted to transmit the data to a processor for analysis. The global positioning sensor allows an operator to determine where the concrete structure has been vibrated.

According to another aspect of the invention, a method of ensuring proper vibration of concrete to remove air therein and promote concrete consolidation includes the steps of providing an apparatus for ensuring proper concrete consolidation. The apparatus includes a global positioning sensor, a recording device, and a transmission device. The method further includes the steps of vibrating the concrete with a vibrator, using the recording device to record data from the vibrating step, using the transmission device to transmit the data to a processor for analysis and determining whether the vibrating step is in compliance, and providing a compliance report to a user.

### BRIEF DESCRIPTION OF THE DRAWINGS

The subject matter that is regarded as the invention may be best understood by reference to the following description taken in conjunction with the accompanying drawing figures in which:

Figure 1 shows an apparatus for quality control according to an embodiment of the invention; and

Figure 2 shows an apparatus for quality control according to an embodiment of the invention.

### DETAILED DESCRIPTION OF THE INVENTION

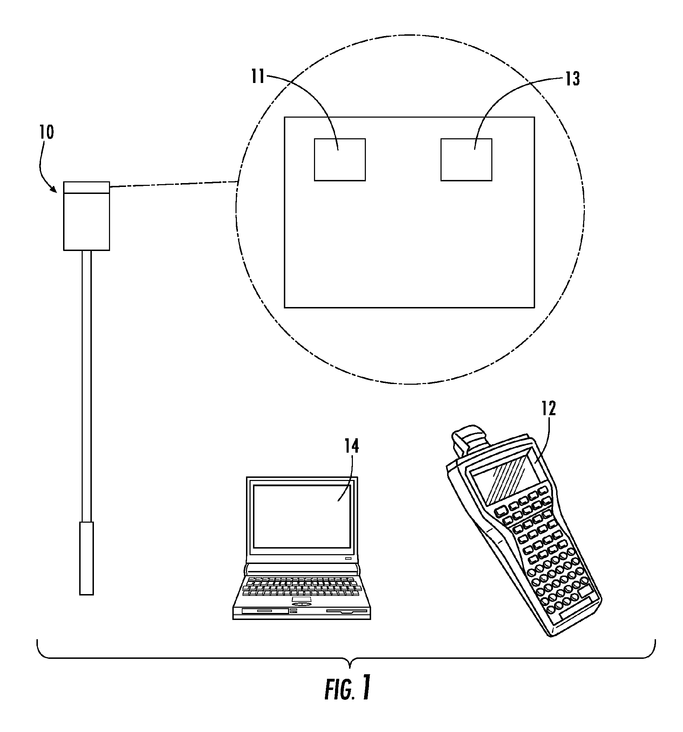

Referring to the drawings, an exemplary apparatus according to an embodiment of the invention is illustrated in Figure 1 and shown generally at reference numeral 10.

The present invention pertains to concrete vibrated with internal vibrators. Internal vibrators do not currently have capabilities for quality control. For instance, there is no track record of where, when, and for how long the concrete vibrated; and there is no way of recording/documenting quality control during this process.

The apparatus 10 may be in the form of a stand alone apparatus attached to a vibrator for quality control of vibration during concrete placement or in the form of a vibrator having built-in quality control. The apparatus 10 records the exact location, timing and duration of the vibration, resulting in a quality control of the vibration process embedded in the vibrator. The apparatus 10 includes a Global Positioning Sensor (GPS) 11; a recording device (data logger) 12 to track time, position, and duration of vibration; a transmission device 13 for wired or wireless capabilities for data transmission to analysis hardware, such as a processor or computer 14 equipped with data analysis software.

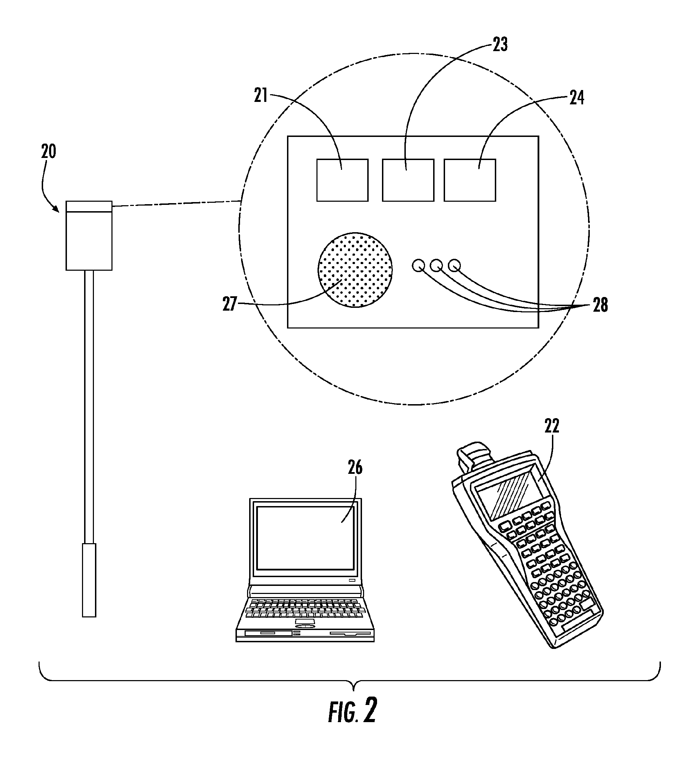

Referring to Figure 2, apparatus 20 may also be in the form of a stand alone apparatus attached to a vibrator for quality control of vibration during concrete placement or in the form of a vibrator having built-in quality control. Like apparatus 10, the apparatus 20 records the exact location, timing and duration of the vibration, resulting in a quality control of the vibration process embedded in the vibrator. The apparatus 20 includes a Global Positioning Sensor (GPS) 21; a recording device (data logger) 22 to track time, position, and duration of vibration; a wired or wireless receiver 23; a transmission device 24 for wired or wireless capabilities for data transmission to analysis hardware, such as a processor or computer 26 equipped with data analysis software; an audible device 27 to provide audible signals representative of the vibration process; and visual indicators, such as LEDs 28, to provide a visual indicator to a user.

The data recorded by apparatuses 10 and 20 may be processed in one of two ways. The first option ensures a real-time follow-up of vibration but only provides a post-analysis of performed vibration. The second option ensures a real-time follow-up of vibration and real time warnings to ensure a proper consolidation of the fresh concrete. Both options may lead to further inspection (NDE, core drilling) to be eventually taken by the owner prior to the reception of the structure.

A more detailed description of both options follows below.
Option #1 - Quality Assurance process:
   1. Set-up criteria for minimal vibration duration and minimal distance between two vibration spots in compliance with the construction codes and local specifities (reinforcement rate);
   2. Record vibration;
   3. Transfer data to computer;
   4. Analyze data and locate non-compliances; and
   5. Provide an automatic report.
Option #2 - Quality Assurance process:
   1. Set-up criteria for minimal vibration duration and minimal distance between two vibration spots in compliance with the construction codes and local specifities (reinforcement rate);
   2. Record vibration;
   3. Real-time transfer to computer, real-time analysis. If non-compliances detected (duration, distance) send signal to vibrating operator for correction;
   4. After finishing vibration of the concrete element or a set of elements, analyze data and locate remaining non-compliances; and
   5. Provide an automatic report.

The foregoing has described an apparatus and method for controlling the quality of concrete. While specific embodiments of the present invention have been described, it will be apparent to those skilled in the art that various modifications thereto can be made without departing from the spirit and scope of the invention. Accordingly, the foregoing description of the preferred embodiment of the invention and the best mode for practicing the invention are provided for the purpose of illustration only and not for the purpose of limitation.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. An apparatus (10, 20) for ensuring proper concrete consolidation, comprising:
(a) a global positioning sensor (11, 21) adapted to provide a location of a vibrator relative to a concrete structure, the vibrator being adapted to vibrate the concrete to remove air contained therein and promote concrete consolidation;
(b) a recording device (12, 22) adapted to record data during vibration of the concrete; and
(c) a data transmission device (13, 24) adapted to transmit the data to a processor (14, 26) for analysis of the data to determine if the vibration is in compliance.

2. The apparatus (10, 20) according to claim 1, wherein the data is selected from the group consisting of time, position, and duration of vibration.

3. The apparatus (10, 20) according to claim 1 or claim 2, wherein the apparatus (10, 20) is connected to the vibrator.

4. The apparatus (10, 20) according to any preceding claim, wherein the data transmission device (13, 24) transmits data wirelessly to the processor (14, 26).

5. The apparatus (10, 20) according to any one of claims 1 to 3, wherein the data transmission device (13, 24) is wired to the processor (14, 26) to allow transmission of the data.

6. The apparatus (10, 20) according to any preceding claim, wherein the processor (14, 26) is a computer having data analysis software installed thereon.

7. The apparatus (20) according to any preceding claim, further including a receiver (23) for receiving a signal from the processor (14, 26), wherein the signal tells an operator of the vibrator whether the vibration is in compliance.

8. The apparatus (20) according to claim 1, further including an audible device (27) adapted to provide an audible signal to an operator of the vibrator indicative of the vibration process.

9. The apparatus (20) according to claim 1, further including a visual indicator (28) adapted to provide a visual indication to an operator of the vibrator indicative of the vibration process.

10. The apparatus (10, 20) according to any preceding claim, further comprising:
(a) a vibrator for vibrating concrete to promote proper concrete consolidation;
(b) a quality control unit comprising the apparatus of claim 1 connected to the vibrator.

11. A method of ensuring proper vibration of concrete to remove air therein and promote concrete consolidation, comprising the steps of:
(a) providing an apparatus (10, 20) for ensuring proper concrete consolidation, having:
(i) a global positioning sensor (11, 21);
(ii) a recording device (12, 22); and
(iii) a transmission device (13, 24);
(b) vibrating the concrete with a vibrator;
(c) using the recording device (12, 22) to record data from the vibrating step;
(d) using the transmission device (13, 24) to transmit the data to a processor (14, 26) for analysis and determining whether the vibrating step is in compliance; and
(e) providing a compliance report to a user.

12. The method according to claim 11, further including the step of setting up criteria related to minimal vibration duration and minimal distance between vibration locations for the processor (14, 26) to compare the data to in determining compliance.

13. The method according to claim 11 or claim 12, wherein the data includes vibrator location during vibration step and duration of vibration step.

14. The method according to any one of claims 11 to 13, wherein the data is transmitted in real-time to provide a user real-time analysis to allow the user to correct any compliance issues.

15. The method according to any one of claims 11 to 14, further including the steps of:
(a) providing a receiver (23); and
(b) using the receiver (23) to receive non-compliance signals from the processor (14, 26), wherein the non-compliance signals alert a user in real-time to allow the user to correct any compliance issues.
